Europäisches Patentamt

(19) European Patent Office

Office européen des brevets

(11) Publication number: **0 220 304 B1**

(12) **EUROPEAN PATENT SPECIFICATION**

(45) Date of publication of patent specification :
24.07.91 Bulletin 91/30

(51) Int. Cl.⁵ : **A61B 17/22**

(21) Application number : **86903732.5**

(22) Date of filing : **23.04.86**

(86) International application number :
**PCT/US86/00886**

(87) International publication number :
**WO 86/06269 06.11.86 Gazette 86/24**

(54) **USE OF LASERS TO BREAK DOWN OBJECTS.**

(30) Priority : **24.04.85 US 726472**

(43) Date of publication of application :
**06.05.87 Bulletin 87/19**

(45) Publication of the grant of the patent :
**24.07.91 Bulletin 91/30**

(84) Designated Contracting States :
**AT BE CH DE FR GB IT LI LU NL SE**

(56) References cited :
EP-A- 0 144 764
EP-A- 0 152 766
EP-A- 0 153 847
WO-A-85/00510
WO-A-85/03631
DE-A- 2 412 690
DE-A- 2 538 960
US-A- 4 207 874
Lasers in Surgery and Medicine, Volume 5,
No. 2, April 1985, pages 160 (abstract no. 82)
and 189 (abstract no. 163)
Surgery, vol. 90, no. 1, July 1981, pages
120-122; Kazuo Orii et
al.:"Choledocholithotomy by Yag laser with a
choledochofiberscope"
The second British conference on lasers in
medicine and surgery, Dec. 1983, pages 1-4;
G.M.Watson:"Laser fragmentation of renal
and biliary calculi"
Medical Instrumentation, vol. 12, no. 2,
March/April 1978, pages 100-105; H.D.Fair:"In
vitro destruction of urinary calculi by laser-
induced stress waves"
The British Journal of Urology, 1983, Vol. 55,
Pages 613-616; G.M.Watson et al.:"Laser
Fragmentation of Renal Calculi"

(56) References cited :
The Journal of Urology, 1968, Vol. 99, Pages
112-114; W.P.Mulvaney et al.:"The Laser
Beam in Urology"
Biomedizinische Technik, vol. 30, no. 7/8,
July/August 1985 (Berlin, DE), Schmidt-Kloiber
et al.: "Laserinduced shock-wave lithotripsy
(LISL)", pages 173-181

(73) Proprietor : **CANDELA LASER CORPORATION**
**19 Strathmore Road**
**Natick, MA 01760 (US)**
Proprietor : **THE GENERAL HOSPITAL**
**CORPORATION**
**55 Fruit Street**
**Boston MA 02114 (US)**

(72) Inventor : **FURUMOTO, Horace**
**14 Woodridge Road**
**Wellesley, MA 02181 (US)**
Inventor : **WATSON, Graham**
**60 Lysias Road**
**London SW12 8BT (GB)**

(74) Representative : **Holdcroft, James Gerald, Dr.**
**et al**
**Graham Watt & Co., Riverhead**
**Sevenoaks, Kent TN13 2BN (GB)**

EP 0 220 304 B1

## Description

Background

This invention relates to a pulsed laser apparatus using a laser beam delivered via an optical fiber to break down a calculus, stone or calcified tissue or other material for removal from within the human body.

Frequently such calculi, stones, or calcified tissue are located in positions which can be reached using only small diameter endoscopes and the optical fiber must be fine enough to pass via the endoscope. The stones are typically in close proximity to heathly tissue.

A pulsed laser apparatus as defined in the first part of Claim 1 is known from US-A- 4 418 688.

Summary of the Invention

The general feature of the invention is in delivering via the optical fiber laser pulses having a wavelength, energy, intensity and pulse duration as defined in the second part of Claim 1, which will break down the calculs, stone, calcified tissue or other material into smaller particles without delivering energy sufficient to cause damage to other tissue in the same vicinity.

The material to be removed from the body is illuminated in a localized region with a pulsed laser. By surrounding the material with liquid and illuminating the localized region with light above a threshold intensity level, a shock wave can be generated from the localized region. It is believed that the shock wave fragments the materials beyond the localized region even though heating is limited to the localized region.

Thus, the stone is safely and relatively quickly broken down into easily removed sand-like particles, without melting. Thermal damage to surrounding tissue is limited. The stone and the particles are not propelled into the surrouding tissue. Degradation of the optical fiber by the laser beam is limited. The fiber can be sufficiently small in diameter to be useful with small diameter endoscopes.

The pulses are at wavelengths corresponding to wavelengths for which the object has a relatively shallow depth of penetration.

The preferred embodiments include the following features. Preferably wavelengths between 350 and 550 nanometers are used for urinary calculi (most preferably 251, 504, or 450 nanometers). The laser is preferably of the pulsed dye type for relatively long pulse durations but may be of other types. The pulses have durations of preferably between .05 and 2 microseconds, and the pulse energy is preferably between .005 and .200 joules. The fiber is flexible and has a core diameter preferably between 60 and 600 microns, and more specifically 200 microns. The distal end of the fiber is in contact with the object (a stone) and the interface between them is surrounded by fluid. The laser pulses are applied in brief bursts, preferably greater than 10 hertz, and remaining fragments are broken down by one-shot pulses.

Other advantages and preferred features of the invention will become apparent from the following description of the preferred embodiment.

Drawings

Fig. 1 is a diagram of a system for breaking down unwanted objects or tissue.

Fig. 2 is a family of curves showing fragmentation thresholds versus pulse energy for different pulse durations.

Fig. 3 is a family of curves indicative of fragmentation threshold pulse energy vesus wavelength for different types of stones.

Fig. 4 is a family of curves indicative of fragmentation threshold pulse energy versus fiber area for different types of stones.

Fig. 5 is a curve indicative of fragmentation threshold pulse energy versus pulse repetition rate.

Fig. 6 is a diagram of pulse timing.

Description of the Preferred Embodiments

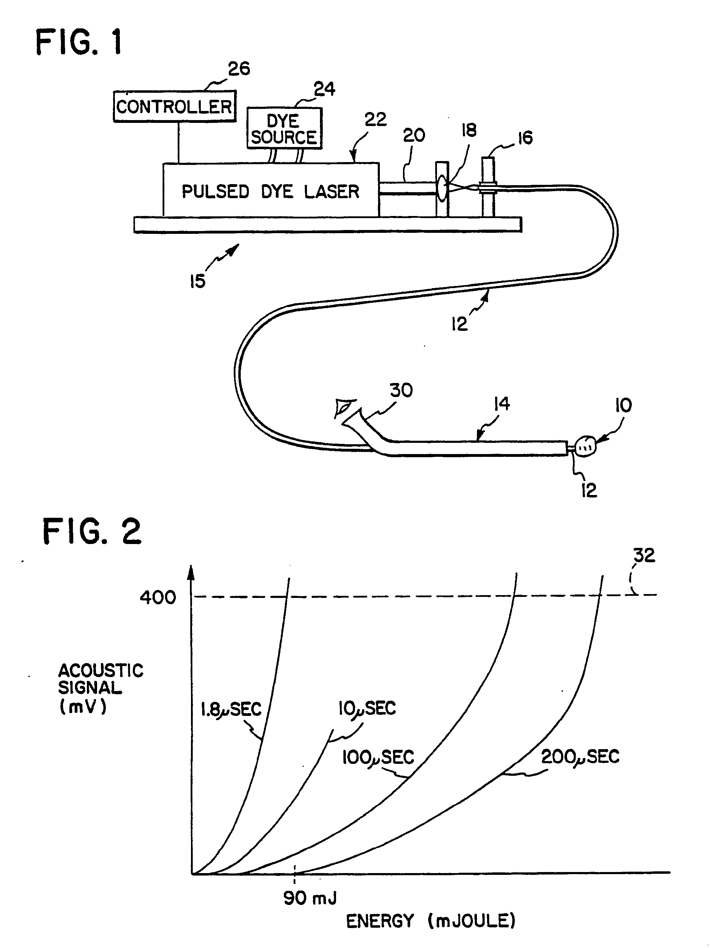

Referring to Fig. 1, a urinary calculus (stone) 10 to be removed from within a human body is contacted by the cleaved distal face of a flexible quartz silica optical fiber 12 (Superguide series available from Fiberguide Industries) having a core diameter in the range of 60 to 400 microns. Fiber 12 passes through a ureteroscope 14 and extends to a laser source 15, where the proximal face of fiber 12 is held in a fiber mount 16 (Model FP2 available from Newport Corporation). The proximal face of fiber 12 is exposed to receive, via a convergent lens 18, (of appropriate focal length for the fiber), a beam 20 from a linear flash lamp pumped pulsed dye laser 22.

EP 0 220 304 B1

Laser 22 is connected to a source 24 of dye of a selected wavelength characteristic. Laser 22 is also connected to a controller 26 which includes a control panel to permit the user to activate and deactivate the laser and to vary the pulse energy and pulse repetition rate of the laser beam. Laser 22 and controller 26 are available from Candela Corporation, Natick, Massachusetts.

Ureteroscope 14 includes an eyepiece 30 through which the user can observe the stone and the distal end of the fiber, as well as a light source (not shown) to illuminate the distal end for viewing and an irrigation lumen to deliver an irrigant to the distal end.

The wavelength at which the laser will be operated (and hence the dye to be used) is chosen in part on the basis of the percentage transmission characteristics of the stone material. For example, the percentage transmission of calcium phosphate and calcium oxalate stone materials for different wavelengths was measured experimentally (by conventional spectroscopy) on sections of dry stones which were sanded to form progressively thinner wafers. The resulting graph of the log of the percentage transmission against thickness was linear indicating the following l/e depths of penetration for different wavelengths.

## 1/e Depth of Penetration (mm)

| Wavelength (nm) | Calcium Phosphate | Calcium Oxalate |
|---|---|---|
| 1064 | 2.16 + 0.8 | 3.58 + 0.85 |
| 577 | 0.81 + 0.2 | 0.50 + 0.1 |
| 504 | 0.54 + 0.05 | 0.30 + 0.2 |
| 450 | 0.42 + 0.05 | 0.24 + 0.05 |
| 308 | 0.25 + 0.03 | 0.18 + 0.1 |

The penetration depth decreases with shorter wavelengths. The smallest penetration depth is the most desirable from the point of view of enabling a low energy threshold to accomplish fragmentation, producing small-size fragments, and limiting the propulsion of fragments into surrounding tissue. Very short wavelengths (shorter than 350 nm) in the ultraviolet range (for example 308 nm), however, are known to be mutagenic and are difficult to deliver via the optical fiber and therefore are avoided. Wavelengths in the range 450 to 550 nanometers are preferred. Dyes are available which operate at the 450 nm (blue) and 504 nm (green) wavelengths. The 450 nm dye fades fairly rapidly. Where the cost of the dye is an issue, the best choice is the 504 nm dye.

The duration of each pulse delivered by the laser is chosen to minimize the energy delivered to the stone while still accomplishing fragmentation (i.e., the breaking down of the stone into smaller particles). Referring to Fig. 2, the threshold energy in millijoules per pulse required to initiate fragmentation of an oxalate stone for a given pulse duration at 577 nm using a 600 micron optical fiber was determined experimentally by measuring an acoustic signal in the stone resulting from the pulse. The acoustic signal was measured electronically in millivolt units. Dashed line 32 represents the acoustic level (nominally 400 millivolts) which corresponds to the initiation of fragmentation in a stone. Each curve represents, for a given pulse duration, the variation of acoustic signal with energy per pulse. The point at which each curve crosses line 32 is the threshold pulse energy level at which fragmentation will occur. The threshold energy level decreases with decreasing pulse duration. Because lower energy pulses are less likely to cause thermal damage or to propel the stone or the broken off particles into surrounding tissue, pulse durations of less than 10 microseconds, preferably between .05 and 2.0 microseconds, are used.

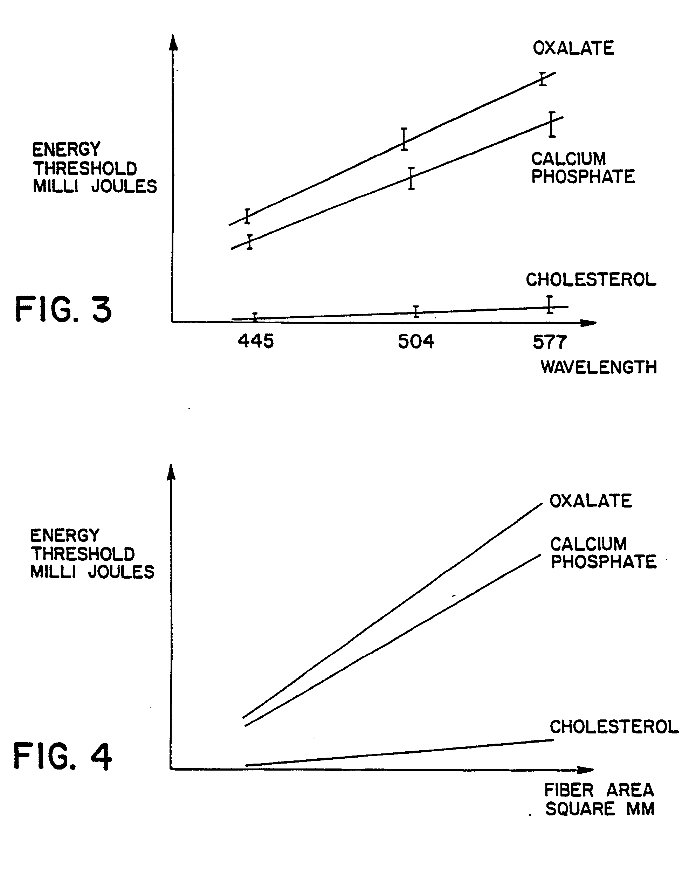

Referring to Fig. 3, the pulse energy threshold for causing an acoustic signal at a given level (25 millivolts) was determined for three different stone types at three different wavelengths, further confirming the desirablility of using shorter wavelengths regardless of the stone material.

Referring to Fig. 4, the relatationship between cross-sectional area of fiber 12 and the threshold pulse energy required to cause an acoustic signal at the 25 mv level was determined for three different stone materials. In all cases the threshold pulse energy decreases linearly with fiber area. Different fiber sizes have been used : 1000, 600, 400, 200, 100 and 60 microns.

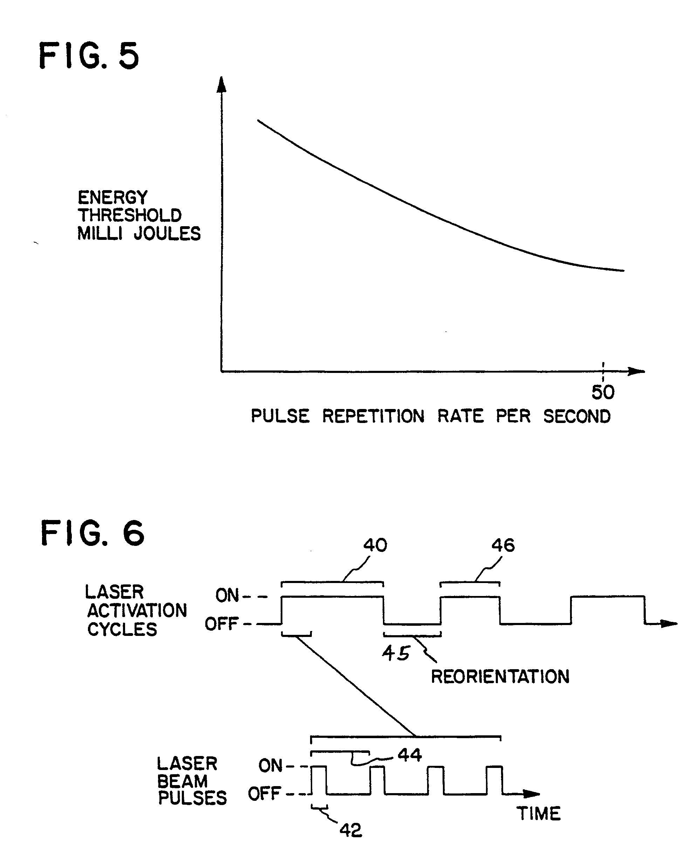

The pulse repetition rate may also be chosen to reduce the threshold pulse energy at which fragmentation is expected to occur. Referring to Fig. 5, the energy required to produce the 25 mv acoustic signal decreased with increasing pulse repetition rate. Furthermore, at higher repetition rates, the fragmentation proceeds more rapidly. At higher repetition rates, however, the dye is depleted faster and the optical fiber is less capable of passing the energy to the stone. A maximum practical rate is not much greater than 100 Hertz and the optimum

3

rate is about 20 Hertz.

It can be shown experimentally that above the energy pulse threshold, the average weight of fragments yielded per pulse increased sharply and that laminated oxalate stones have a substantially lower fragmentation threshold than do homogeneous oxalate stones. Thus the pulse energy can be varied to break down different stones.

## Operation

In operation, after inserting the ureteroscope 14 to reach the site at which the stone 10 is located, the distal end of the fiber 12 is inserted through the ureteroscope and oriented by sight, so that the distal face of the fiber is in contact with the stone 10. The site is irrigated via a lumen in the ureteroscope so that the store is surrounded by liquid. The laser is set at a wavelength between 450 and 550 nanometers by selecting an appropriate dye. The pulsed dye laser controller 26 is adjusted to set the pulse energy and pulse repetition rate. The laser pulse energy is initially set at a value which is lower than the threshold fragmentation level and then increased until the desired fragmentation effect has been attained. Preferably the pulsed dye laser is operated at about 30 millijoules per pulse for a 200 micron fiber and about 100-150 millijoules per pulse for a 600 micron fiber, and in no event more than about 200 millijoules. The pulse repetition rate is set between 10 and 50 Hertz.

Referring to Fig. 6, the laser 22 is activated for a brief burst, for example for a period of a second or a fraction of a second (identified as 40 in Fig. 6). The laser pulse duration 42 is typically between 0.1 and 2.0 microseconds. The period of the pulse repetition is identified as 44 on Fig. 6. During activation period 40, a portion of the stone breaks down into a combination of vapor and sand-like particles small enough to be easily removed.

The distal face of the fiber is then reoriented (during a period identified as 45 in Fig. 6) to be once again in contact with the stone. Then the laser is again activated briefly for a burst period 46, to cause another portion of the stone to break down. The process is repeated until the entire stone has been broken down. Any fragments which need to be broken down further can then be broken down by touching them with the distal end of the fiber and applying a single shot laser pulse.

The stone is safely and relatively quickly broken down into easily removed sand-like particles, without melting. Thermal damage to surrounding tissue is limited. The stone and particles are not propelled into the surrounding tissue. Degradation of the optical fiber by the laser beam is limited. The fiber can be sufficiently small in diameter to be useful with small diameter endoscopes.

Other embodiments are within the following claims. For example, although less desirable, laser 22 can be an excimer laser tuned to a particular wavelength by a selected gas mixture. The wavelength is chosen to be as short as possible while still permitting the pulses to be delivered via the optical fiber. Preferably the gas mixture is xenon-fluoride providing a wavelength of 351 nm. The resulting pulses have very shallow penetration into the stone and operate to break the stone into extremely fine particles and vapor. Progress through the stone is slower per pulse than for the pulsed dye laser, but this is offset by higher pulse repetition rates which are possible with the excimer laser. The pulse durations typical of excimer lasers are 10 nanoseconds but can be lengthened by various techniques to 80 or more nanoseconds. Such pulse durations make the pulses somewhat more difficult to deliver via the optical fiber than the pulsed dye laser pulses.

Using the excimer laser on an oxalate calculus via a 1000 micron fiber at 351 nm, a repetition rate of 200 Hertz, and a pulse energy of 30 millijoules (energy density of 1.6 joules/cm$^2$), produced an average yield of fragments per pulse of 10 micrograms. By comparison, using the pulsed dye laser at 450 nm and a pulse energy of 20 joules/cm$^2$ via a 600 micron fiber generated 100 micrograms of fragments per pulse. Using the pulsed dye laser at 504 nm at a pulse energy of 25 joules/cm$^2$ via a 600 micron fiber yielded 1 milligram per pulse.

The product of breaking down the stone is about 90% vapor for the excimer laser and 10% for the pulsed dye laser.

In other embodiments, gallstones or arterial plaque may be broken down by a pulsed dye laser at 450 nm, and any appropriate technique for reaching the stone with the distal end of the fiber may be used.

The following describes observations made in the process of breaking down calculus, stone and calcified tissue using a laser.

1. Laser radiation is absorbed by the target material at wavelengths characteristic of its absorption spectra. Laser light of shorter wavelengths is better for white or translucent material.

2. The laser intensity must be greater than a certain level before a significant effect is observed. The intensity is proportional to the energy delivered and inversely proportional to the pulse duration of the laser, and the distal end of the fiber must touch or be in close proximity to the target for maximum effect. However, a fiber is not necessary inasmuch as the same effect can be obtained by focusing the laser beam on the target so that the intensity threshold is reached.

3. When the threshold for breakdown is reached, a loud acoustic signal is heard. The target material must be completely immersed in liquid for maximum breakdown. A wetted target or a target slightly below the surface of the liquid (2 or 3 mm) will give a loud acoustic signal but will not readily break down the target. It is believed that the fragmentation process is as follows :

1. The laser radiation is first absorbed by the target. For white or translucent material, shorter wavelengths are preferred.

2. A minimum laser threshold intensity (power per unit area) is necessary to vaporize, heat and ionize the target.

3. The laser energy is confined in a small volume by a surrounding liquid. Increasing the energy density as more laser light is absorbed increases the pressure in the volume to several hundred kilobars. At such pressures, shock waves with average velocities over 0.5 mm per microsecond can be formed. "Laser Induced High Pressure Shock Waves in Water", C.E. Bell and J.A. Landt, Applied Physics Letters 10, 46 (1966) and "Intense Ruby Laser Induced Acoustic Impulses", E.F. Carame, C.E. Moeller and N.A. Clark, Jrn of Acoustical Soc of America 40, 1463 (1966).

4. The spherical shock wave is propagated into the calculus, stone or calcified tissue to break it down. The liquid is necessary to confine the interaction volume so that a high pressure shock is generated. The liquid also helps to couple the shock wave into the target. If the target is only wetted or submerged slightly, the interaction volume expands so that only a moderate to weak shock is generated which will not break down the target.

There are several considerations in the optimization of the use of lasers to break down objects for removal from within the body. They are energy per pulse, intensity (power density), pulse duration, repetition rate, color, fiber size, and fiber damage level. These considerations are not independent of each other. In addition, treatment time should be shortened whenever possible and risk of damage to living tissue in the body should be minimized.

1. Energy per pulse. Energy per pulse should be as large as possible to maximize the amount of material to be broken down in the shortest period of time.

2. Fiber size. Small fibers are preferred because they are more flexible and because endoscopes or catheters composing the delivery systems can be made smaller. Fibers over 600 microns in diameter are too large, while fibers smaller than 60 microns will not be able to transmit much total power or energy, thereby increasing treatment time. One to two hundred micron fibers are considered optimum.

3. Intensity. The laser intensity out of the fiber must be high enough to form a shockwave at the taget. An intensity of at least 10 megawatts per square centimeter is desired. The area of the spot is determined by the fiber cross section. The highest intensity occurs when the fiber touches the target and intensity is quickly reduced as the fiber is withdrawn from contact. A lens system to focus the light from the fiber on to the target to obtain the desired intensity is conceivable but difficult to reduce to practice. Furthermore, the shock generated erodes the fiber tip. This is not too serious for the distal surace of the cleaved fiber tip but is a serious effect for a lens unless the lens is far removed from the focus.

4. Pulse duration. Total power for a given energy can be increased by reducing pulse duration. Typical Q switched, excimer or nitrogen lasers will have pulse durations in the order of 1 to 20 nsec. The damage intensity for quartz fiber is about 300 to 400 megawatts per square cm. A 400 micron fiber will transmit approximately 10 mj in a 20 nsec pulse at the damage level provided that the laser uniformly illuminates the fiber. However, the laser beam must be focused into the fiber and peak intensity spots will limit the total energy to a few millijoules. Higher energy throughput is desired for rapid treatment.

Longer pulses of at least .05 microseconds, and preferably greater than the .1 microseconds available with dye lasers, allow more energy to be transmitted for a given fiber size. However, too, long a pulse will allow the interaction volume to expand and the shock to dissipate. For high pressure shocks in liquids, a pulse duration 2 microseconds or less is desired.

5. Repetition rate. The higher the setting of the repetition rate, the faster is the treatment. However, single shot capacity is also necessary to further fragment small broken off particles.

Table I sets forth the optimized operating ranges for various treatments. A flashlamp excited dye laser can be designed to perform at the optimum conditions for each of the listed treatments.

A less desirable embodiment is the use of an excimer, gold or copper vapor, frequency doubled repetitively switched YAG or a nitrogen laser for laser 22. These lasers have higher peak output powers and shorter pulse durations than a flashlamp excited dye laser and the fiber damage threshold of 400 MW/cm$^2$ is reached at relatively low energy. The higher intensity requires the use of larger diameter fiber to prevent damage to the fiber and allows for even larger diameter fibers while still providing the threshold intensity. The material is not fragmented substantially beyond the illuminated region. The fragments which are generated are much finer than those obtained with the long pulse laser. The amount of material removed per pulse for a given fiber size is much lower for the high peak power laser than for the pulsed dye laser, so high repetition rates should be used.

# TABLE I

## Optimized Operating Range for a Pulsed Laser

|  | Urinary Calculi | Gallstones | Calcified Tissue |
|---|---|---|---|
| Fiber Diameter (low) | 60 microns | 60 microns | 60 microns |
| Fiber Diameter (high) | 600 microns | 600 microns | 600 microns |
| Fiber Diameter (optimum) | 200 microns | 200 microns | 200 microns |
| Energy/Pulse (low) | 5 mj | 5 mj | 5 mj |
| Energy/Pulse (high) | 100 mj | 100 mj | 200 mj |
| Pulse Duration | .05 to 2 $\mu$sec | .05 to 2 $\mu$sec | .05 to 2 $\mu$sec |
| Rep Rate (single shot) | Desireable | Desireable | Desirable |
| Rep Rate (optimum) | 20 Hz | 20 Hz | 20 Hz |
| Intensity (low) | 5 MW/cm² | 5 MW/cm² | 5 MW/cm² |
| Intensity (high) | 400 MW/cm² | 400 MW/cm² | 400 MW/cm² |

Table II of optimized operating ranges has been assembled for the high peak power lasers.

EP 0 220 304 B1

## TABLE II

### Operating Range for a Low Energy but High Peak Power Pulsed Laser

|  | Urinary Calculi | Gallstones | Calcified Tissue |
|---|---|---|---|
| Fiber Diameter (low) | 200 microns | 200 microns | 200 microns |
| Fiber Diameter (high) | 1000 microns | 1000 microns | 1000 microns |
| Fiber Diameter (optimum) | 600 microns | 600 microns | 600 microns |
| Peak Power for 600 Micron fiber (high) | 1 MW | 1 MW | 1 MW |
| Pulse Duration | prefer 50-100 nsec | prefer 50-100 nsec | prefer 50-100 nsec |
| Rep Rate | 100 Hz | 100 Hz | 100 Hz |
| Intensity (low) | 5 MW/cm$^2$ | 5 MW/cm$^2$ | 5 MW/cm$^2$ |
| Intensity (high) | 400 MW/cm$^2$ | 400 MW/cm$^2$ | 400 MW/cm$^2$ |

## Claims

1. Pulsed laser apparatus adapted for fragmentation of target material (10) within a body, the apparatus comprising a pulsed laser (22) and an optical fiber delivery apparatus, the delivery apparatus comprising an optical fiber (12) of diameter less than 1000 µm through which a localized region of the target material is illuminated in the operation of the apparatus and irrigation means for irridating a site within the body where the taget material is located characterized in that :

the laser produces laser pulses of duration of at least 0.05 microseconds of energy less than 200 millijoules and of intensity at the localized region greater than 10 megawatts per square centimeter, and the laser and delivery apparatus are adapted to directly illuminate the target material with light of wavelengths characteristic of the absorption spectra of the target material to provide sufficiently shallow depth of penetration, so that at least some fraction of the target material is fragmented and the surrounding non-targeted tissue is not damaged by a shockwave triggered through application of said pulsed laser light, said shockwave being sufficient to cause an acoustic signal within the illuminated target.

2. Apparatus as claimed in claim 1, characterized in that the pulsed laser is capable of producing and delivering an energy per pulse between 5 millijoules and 200 millijoules.

3. Apparatus as claimed in claim 1 or claim 2, characterised in that the pulsed laser is arranged for a pulse duration of at least 0.1 microseconds.

4. Apparatus as claimed in any preceding claim, characterised in that the pulse duration is less than 10 microseconds.

5. Apparatus as claimed in claim 4, characterised in that the pulse duration is between 0.1 and 2 microseconds.

6. Apparatus as claimed in any preceding claim, characterised in that the pulsed laser is capable of a pulse repetition rate of greater than 10 Hertz.

7. Apparatus as claimed in any preceding claim, characterised in that the pulsed laser is a dye laser.

8. Apparatus as claimed in any preceding claim, characterised in that the laser light has a wavelength between 350 and 628 nanometers.

9. Apparatus as claimed in any preceding claim, characterised in that the laser light has a wavelength between 350 and 550 nanometers.

10. Apparatus as claimed in any preceding claim, characterised in that the optical fiber has a diameter between 60 and 600 microns.

## Patentansprüche

1. Impulslasergerät für eine Fragmentierung von Zielmaterial (10) innerhalb eines Körpers, bestehend aus einem Impulslaser (22) und einem Zuführgerät für eine optische Faser, wobei das Zuführgerät eine optische Faser (12) mit einem Durchmesser von weniger als 100 µm, durch die eine lokalisierte Zone des Zielmaterials im Betrieb des Gerätes beleuchtet wird, und eine Spülungseinrichtung zum Spülen einer Stelle innerhalb des Körpers umfaßt, an der das Zielmaterial lokalisiert ist, dadurch gekennzeinchnet, daß

der Laser Laserimpulse mit einer Dauer von zumindest 0,05 Mikrosekunden mit einer Energie von weniger als 200 Millijoule und mit einer Intensität in der lokalisierten Zone von mehr als 10 Megawatt pro Quadratzentimeter erzeugt und der Laser und das Zuführgerät für eine direkte Beleuchtung des Zielmaterials mit Licht von für die Absorptionsspektren des Zielmaterials charakteristischen Wellenlängen zur Erzeugung einer ausreichend flachen Eindringungstiefe geeingnet sind, so daß zumindest ein Stück des Zielmaterials fragmentiert und das umgebende, nicht-angezielte Gewebe nicht beschädigt wird durch eine Stoßwelle, die durch Anwendung des Impulslaserlichts ausgelöst wird, wobei die Stoßwelle so groß ist, daß ein akustishes Signal innerhalb des beleuchteten Ziels hervogerufen wird.

2. Gerät nach Anspruch 1, dadurch gekennzeichnet, daß der Impulslaser in der Lage ist, eine Energie pro Impuls zwischen 5 Millijoule und 200 Millijoule zu erzeugen und zu liefern.

3. Gerät nach Anspruch 1 oder Anspruch 2, dadurch gekennzeichnet, daß der Impulslaser für eine Impulsdauer von zumindest 0,1 Mikrosekunden ausgelegt ist.

4. Gerät nach einem beliebigen vorhergehenden Anspruch, dadurch gekennzeichnet, daß die Impulsdauer weniger als 10 Mikrosekunden beträgt.

5. Gerät nach Anspruch 4, dadurch gekennzeichnet, daß die Impulsdauer zwischen 0,1 und 2 Mikrosekunden liegt.

8

6. Gerät nach einem beliebigen vorhergehenden Anspruch, dadurch gekennzeichnet, daß der Impulslaser zu einer Impulswiederholungsrate von mehr als 10 Hertz in der Lage ist.

7. Gerät nach einem beliebigen vorhergehenden Anspruch, dadurch gekennzeichnet, daß der Impulslaser ein Farbstofflaser ist.

8. Gerät nach einem beliebigen vorhergehenden Anspruch, dadurch gekennzeichnet, daß das Laserlicht eine Wellenlänge zwischen 350 und 626 Nanometern besitzt.

9. Gerät nach einem beliebigen vorhergehenden Anspruch, dadurch gekennzeichnet, daß das Laserlicht eine Wellenlänge zwischen 350 und 550 Nanometern besitzt.

10. Gerät nach einem beliebigen vorhergehenden Anspruch, dadurch gekennzeichnet, daß die optische Faser einen Durchmesser zwischen 60 und 600 Mikron besitzt.


**Revendications**

1. Appareil à laser pulsé adapté à la fragmentation d'un matériau cible (10) dans un corps, cet appareil comprenant un laser pulsé (22) et un appareil de transmission à fibre optique, cet appareil de transmission comprenant une fibre optique (12) d'un diamètre inférieur à 1000 micromètres, à travers laquelle une région localisée du matériau cible est irradié lors du fonctionnement de l'appareil, et des moyens d'irigation pour irriguer un endroit dans le corps où se trouve le matériau cible, caractérisé en ce que le laser produit des impulsions laser d'une durée d'au moins 0,05 microseconde, ayant une énergie inférieure à 200 millijoules et une intensité, à l'endroit de la région localisée, supérieure à 10 mégawatts par centimètre carré, et le laser et l'appareil de transmission sont adaptés de manière à irradier directement le matériau cible avec une lumière ayant une caractéristique de longueur d'onde du spectre d'absorption du matériau cible pour assurer une profondeur de pénétration suffisamment faible, de telle façon qu'au moins une fraction du matériau cible soit fragmentée et que le tissu environnant, non visé, ne soit pas endommagé par une onde de choc provoquée par l'application de la lumière laser pulsée, cette onde de choc étant suffisante pour produire un signal acoustique dans la cible irradiée.

2. Appareil suivant la revendication 1 caractérisé en ce que le laser pulsé est capable de produire et de transmettre une énergie par implusion comprise entre 5 millijoules et 200 millijoules.

3. Appareil suivant la revendication 1 ou 2 caractérisé en ce que le laser pulsé est agencé de manière à avoir une durée d'impulsion d'au moins 0,1 microseconde.

4. Appareil suivant l'une quelconque des revendications précédentes caractérisé en ce que la durée d'impulsion est inférieure à 10 microsecondes.

5. Appareil suivant la revendication 4 caractérisé en ce que la durée d'impulsion est comprise entre 0,1 et 2 microsecondes.

6. Appareil suivant l'une quelconque des revendications précédentes caractérisé en ce que le laser pulsé est capable d'émettre des impulsions à une cadence de répétition supérieure à 10 Hertz.

7. Appareil suivant l'une quelconque des revendications précédentes caractérisé en ce que le laser pulsé est un laser à colorant.

8. Appareil suivant l'une quelconque des revendications précédentes caractérisé en ce que la lumière du laser a une longueur d'onde comprise entre 350 et 628 nanomètres.

9. Appareil suivant l'une quelconque des revendications précédentes caractérisé en ce que la lumière du laser a une longueur d'onde comprise entre 350 et 550 nanomètres.

10. Appareil suivant l'une quelconque des revendications précédentes caractérisé en ce que la fibre optique a un diamètre compris entre 60 et 600 micromètres.

# FIG. 1

# FIG. 2

**FIG. 3**

ENERGY THRESHOLD MILLI JOULES — OXALATE, CALCIUM PHOSPHATE, CHOLESTEROL — WAVELENGTH (445, 504, 577)

**FIG. 4**

ENERGY THRESHOLD MILLI JOULES — OXALATE, CALCIUM PHOSPHATE, CHOLESTEROL — FIBER AREA SQUARE MM

# FIG. 5

ENERGY
THRESHOLD
MILLI JOULES

50

PULSE REPETITION RATE PER SECOND

# FIG. 6

LASER
ACTIVATION
CYCLES

ON
OFF

40

46

45

REORIENTATION

LASER
BEAM
PULSES

ON
OFF

44

42

TIME